# EUROPÄISCHE PATENTSCHRIFT

(11) **EP 1 093 776 B1**
(45) Veröffentlichungstag und Bekanntmachung des Hinweises auf die Patenterteilung: **01.12.2004**
(21) Anmeldenummer: 00110627.7
(22) Anmeldetag: 18.05.2000
(51) Int. Cl.: A61F 2/38

(54) **Kniegelenkendoprothese**
Knee joint endoprosthesis
Endoprothèse de l'articulation du genou

(30) Priorität: 18.10.1999 DE 19950112
(43) Veröffentlichungstag der Anmeldung: 25.04.2001
(73) Patentinhaber: PLUS Endoprothetik AG, 6343 Rotkreuz (CH)
(72) Erfinder: Hauri, Thomas, 5053 Staffelbach (CH); Hauri, Bernhard, 5053 Staffelbach (CH); Schmotzer, Hans, Dr., 8048 Zürich (CH); Berner, Werner, Dr., 5018 Erlinsbach (CH)
(74) Vertreter: Popp, Eugen, Dr.

(56) Entgegenhaltungen:
- EP-A- 0 529 408
- US-A- 3 824 630

## Beschreibung

Die Erfindung betrifft eine Kniegelenkendoprothese mit einem Tibiateil mit ebener Tibialagerfläche, einem auf dieser in Richtung anterior-posterior verschiebbaren Lagerkörper mit zwei konkav gewölbten Lagerschalen zur beweglichen Aufnahme eines Femurgelenkteils und mit einer Drehführung, die eine Rotation des Lagerkörpers auf der Tibialagerfläche um eine senkrecht auf dieser stehenden Drehachse erlaubt.

Eine derartige Kniegelenkendoprothese ist bekannt zum Beispiel aus der EP 0 529 408 B1 oder EP 0 519 873 B1.

Beide bekannten Konstruktionen stellen Kniegelenkendoprothesen dar, welche nicht nur Gelenkflächenkongruenz und Translationsmöglichkeit eines sogenannten Meniskusknies aufweisen, sondern diese auch bei Rotationsbewegungen aufrechterhalten und weitgehende Luxationssicherheit des Lagerkörpers gewährleisten sowie einen annähernd normalen, physiologischen Bewegungsablauf ermöglichen.

Dabei ist die Konstruktion nach der EP 0 519 873 B1 dadurch gekennzeichnet, daß die Translationsbewegung des Lagerkörpers nicht durch Anschläge begrenzt ist.

Es hat sich jedoch gezeigt, daß dann sehr leicht Weichteilreizungen sowie eine Überbeanspruchung der Kreuzbänder auftreten.

Auch sollte es möglich sein, daß der Chirurg erst in der Schlußphase einer Operation entscheidet, ob er ein Knieimplantat einsetzt, welches entweder Translation und Rotation oder nur Rotation des Lagerkörpers erlaubt. Diese Wahlmöglichkeit hat der Chirurg bei den bekannten Konstruktionen nicht.

Schließlich sollte der Chirurg die Möglichkeit haben, in der Schlußphase der Operation die maximale Translationsbewegung des Lagerkörpers in Richtung posterior-anterior festzulegen.

Dementsprechend liegt der vorliegenden Erfindung die Aufgabe zugrunde, eine Kniegelenkendoprothese der eingangs genannten Art zu schaffen, die nicht zu Weichteilreizungen oder einer Überbeanspruchung der Kreuzbänder führt. Weiterhin sollte diese Konstruktion Überbestimmungen mit der Gleitlagerung zwischen Lagerkörper und Tibialagerfläche weitgehend vermeiden und zwängungsfrei arbeiten. Außerdem sollte die Kniegelenkprothese maximale Freiheitsgrade für eine anatomiegerechte Führung bieten.

Diese Aufgabe wird durch die kennzeichnenden Merkmale des Anspruches 1 gelöst. Dementsprechend ist die erfindungsgemäße Kniegelenkendoprothese dadurch gekennzeichnet, daß die Drehführung des Lagerkörpers einen Lenker umfaßt, der am Lagerkörper relativ zu diesem schwenkbar gehalten und am Tibiateil medial-lateral geführt und/oder drehgeführt ist.

Konstruktive Details der erfindungsgemäßen Kniegelenkendoprothese sind in den Unteransprüchen beschrieben.

In einer zweckmäßigen Ausführung ist der Lenker einerseits am Tibiateil in einer sich in anterior-posterior Richtung langlochartig erstreckenden Ausnehmung medial und lateral geführt. Andererseits ist er am Lagerkörper entweder relativ zu diesem dreh- und schwenkbar gehalten, oder er ist zusammen mit der Ausnehmung (bzw. dem die Ausnehmung realisierenden Bauteil) im Tibiateil um eine senkrecht auf der Tibialagerfläche stehende Drehachse drehbar gelagert.

Eine weiter bevorzugte Ausführungsform ist dadurch gekennzeichnet, daß die langlochartige Ausnehmung auf Höhe der Tibialagerfläche eine sacklochartige Vertiefung begrenzt, die innerhalb eines sich distal erstreckenden Tibiagehäuses ausgebildet ist, und die sich mindestens in ihrem oberen Bereich in Richtung anterior-posterior von distal nach proximal trapezförmig erweitert. In einer weiter bevorzugten Fortbildung ist die Vertiefung auch zu ihrem Boden hin wieder trapezförmig erweitert, so daß der Lenker um eine von seinem unteren Ende beabstandete (in Höhe der engsten Stelle der Vertiefung liegende) Drehachse zwängungsfrei schwenkbar ist.

Medial und lateral ist die sacklochartige Vertiefung durch sich parallel zueinander und etwa senkrecht zur Tibialagerfläche erstreckende Wände begrenzt, deren Abstand voneinander geringfügig größer ist als der Durchmesser des in der sacklochartigen Vertiefung aufgenommenen Lenkers bzw. Lenkerabschnitts. Letzterer soll mit Spielpassung medial und lateral gleitend geführt sein.

Die Schwenklagerung des erfindungsgemäßen Lenkers in Richtung anterior-posterior wird in einer Variante dadurch erreicht, daß der Lenker am proximalen Ende einen kugelartigen Kopf aufweist, der in eine komplementär ausgebildete Schwenklagerausnehmung am Lagerkörper eingreift, insbesondere in diese einrastbar ist. Alternativ kann am proximalen Ende des Lenkers eine sich in Richtung medial-lateral etwa parallel zur Tibialagerfläche erstreckende Schwenkachse vorgesehen sein, die ebenfalls in eine komplementär ausgebildete Schwenklagerausnehmung am Lagergehäuse eingreift.

Die Gleitlagerung des Lenkers am Tibiateil erfolgt vorteilhafterweise innerhalb einer Lagerhülse aus Kunststoff, insbesondere Polyethylen, wobei diese Lagerhülse am Tibiateil rotationsgesichert plaziert ist, insbesondere innerhalb eines sich distal erstreckenden Tibiagehäuses. Damit ist sichergestellt, daß nach der Operation die Gleitführung des Lenkers exakt in Richtung posterior-anterior erhalten bleibt.

In einer speziellen Ausführung ist die langlochartige Ausnehmung zur medialen Seite hin konkav, d. h. zur lateralen Seite hin konvex gekrümmt. Insbesondere hat sie die Form eines Kreisbogens mit einem medial, insbesondere innerhalb der Kontur des Lagerkörpers, liegenden Krümmungsmittelpunkt. Der mit dieser Ausnehmung zusammenwirkende Lenker bzw. Lenkerabschnitt (Lenkerschaft) hat bevorzugt entweder einen kreisförmigen oder einen in Anpassung an die Bogenform der Ausnehmung zwei gekrümmte Wandabschnitte aufweisenden Querschnitt.

Im übrigen weist der Lenker bzw. der in der langlochartigen Ausnehmung aufgenommene Lenkerabschnitt in Anpassung an eine Ausnehmung mit ebenen Wandungsabschnitten entweder ebenfalls einen kreisförmigen Querschnitt, oder sein Querschnitt umfaßt zwei seitliche Halbkreise verbindende Geraden.

Bei einer alternativen Ausführungsform ist der Lenker mit seinem distalen Ende am Tibiateil verschwenkbar gelagert, wobei diese distale und/oder die proximale Schwenklagerung am Lagerkörper eine lenkeraxiale Translationsbewegung erlaubt, so daß eine dauerhafte Gleitauflage des Lagerkörpers auf der Tibialagerfläche unabhängig von der Schwenkstellung des Lenkers gewährleistet ist.

Der Lenker besteht vorzugsweise aus einer körperverträglichen Metalllegierung (Titanlegierung), ähnlich wie das Tibiateil.

Nachstehend werden Ausführungsformen einer erfindungsgemäßen Kniegelenkendoprothese anhand der beigefügten Zeichnung näher beschrieben. Es zeigen:
- Fig. 1: eine erste Ausführungsform einer erfindungsgemäßen Kniegelenkendoprothese (Tibiateil) im Längsschnitt in Richtung posterior-anterior;
- Fig. 2: die Kniegelenkendoprothese gemäß Fig. 1 im Längsschnitt in Richtung medial-lateral;
- Fig. 3: die Kniegelenkendoprothese gemäß den Figuren 1 und 2 in Draufsicht;
- Fig. 4a: einen Teil einer zweiten Ausführungsform einer erfindungsgemäßen Kniegelenkendoprothese im Längsschnitt in Richtung anterior-posterior;
- Fig. 4b: einen Schnitt durch den Lenker gemäß Fig. 4a;
- Fig. 5: die Kniegelenkendoprothese gemäß Fig. 4a im Schnitt in Richtung medial-lateral;
- Fig. 6: eine dritte Ausführungsform einer erfindungsgemäßen Kniegelenkendoprothese im Längsschnitt in Richtung medial-lateral;
- Fig. 7: die Kniegelenkendoprothese gemäß Fig. 6 im Längsschnitt in Richtung posterior-anterior (Schnitt A-A in Fig. 8);
- Fig. 8: die Kniegelenkendoprothese gemäß den Figuren 6 und 7 in Draufsicht;
- Fig. 9: einen Teil einer erfindungsgemäßen Kniegelenkendoprothese im Längsschnitt in Richtung medial-lateral unter Darstellung eines Details zur Festlegung des Lagerkörpers am Tibiateil;
- Fig. 10a bis 10c: eine weitere Ausführungsform der erfindungsgemäßen Kniegelenkendoprothese im Längsschnitt (Ausschnittdarstellung) in Richtung anterior-posterior;
- Fig. 11a und 11b: eine schematische Darstellung einer weiteren Ausführungsform der Kniegelenkendoprothese in einer Draufsicht bzw. im Querschnitt und
- Fig. 12: eine Prinzipskizze zur Erläuterung allgemeiner Realisierungsmöglichkeiten der erfindungsgemäßen Kniegelenkendoprothese (als posterior-anteriorer-Längsschnitt).

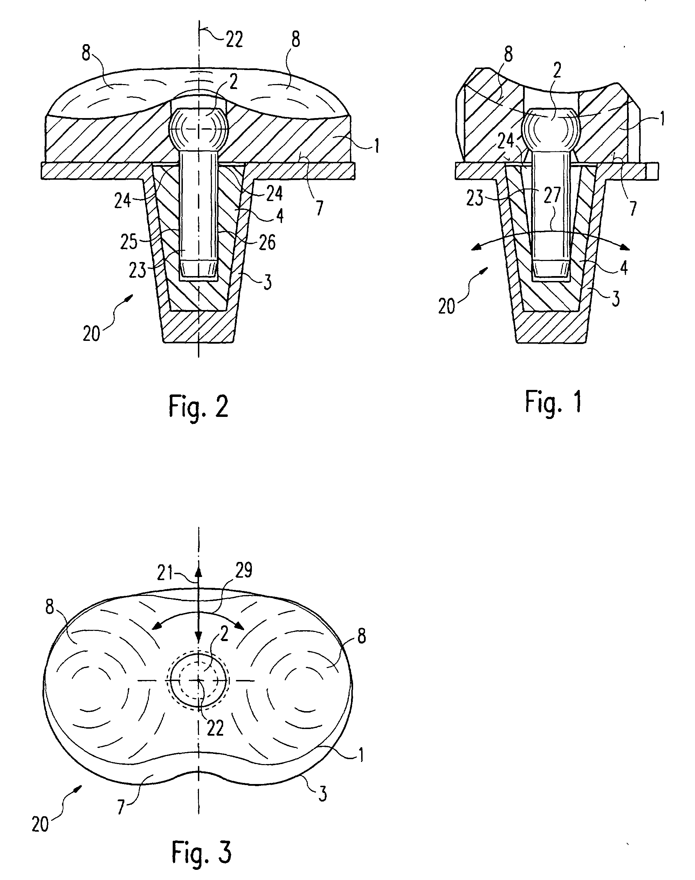

Gemäß den Figuren 1 bis 3 umfaßt eine erste Ausführungsform einer Kniegelenkendoprothese ein Tibiateil 20 mit ebener Tibialagerfläche 7, auf dem ein Lagerkörper 1 in Richtung anterior-posterior verschiebbar ist (Doppelpfeil 21 in Fig. 3). Der Lagerkörper umfaßt zwei konkav gewölbte Lagerschalen 8 zur beweglichen Aufnahme eines nicht dargestellten Femurgelenkteils (Femurschlitten).

Des weiteren ist eine Drehführung für den Lagerkörper 1 vorgesehen, die den Lagerkörper 1 auf der Tibialagerfläche 7 um eine senkrecht auf dieser stehenden Drehachse 22 führt (siehe Doppelpfeil 29 in Fig. 3). Die Drehführung umfaßt einen Lenker 23, der einerseits am Lagerkörper 1 relativ zu diesem sowohl dreh- als auch schwenkbar gehalten und andererseits am Tibiateil 20 bzw. in einem sich distal erstreckenden Gehäuse 3 des Tibiateils innerhalb einer sich in Richtung anterior-posterior erstreckenden langlochartigen Ausnehmung 24 medial und lateral geführt ist. Der Lenker 23 ist bei der dargestellten Ausführungsform als Rundbolzen ausgeführt. Am proximalen Ende weist der Lenker 23 einen kugelartigen Kopf 2 auf, der in eine komplementär ausgebildete Schwenklagerausnehmung am Lagerkörper 1 eingreift, insbesondere von proximal nach distal einrastbar ist.

Die langlochartige Ausnehmung 24 stellt ein Sackloch dar, das sich in Richtung anterior-posterior von distal nach proximal keilartig erweitert (siehe Fig. 1) sowie medial und lateral durch sich parallel zueinander und etwa senkrecht zur Tibialagerfläche 7 erstreckende Wände 25, 26 begrenzt ist, deren Abstand voneinander geringfügig größer ist als der Durchmesser des in das Sackloch 10 eingetauchten Lenkers 23 bzw. entsprechenden Lenkerabschnitts. Der Lenker 23 ist mit Spielpassung zwischen den Begrenzungswänden 25, 26 gleitgelagert.

Durch die erwähnte trapezförmige bzw. keilartige Erweiterung des Sackloches ist eine Kippbewegung des Lenkers 23 innerhalb der Ausnehmung 24 in Richtung anterior-posterior entsprechend dem Doppelpfeil 27 in Fig. 1 möglich. Dementsprechend läßt sich auch der Lagerkörper in Richtung posterior-anterior auf der Tibialagerfläche 7 parallel verschieben.

Die Gleitlagerung des Lenkers 23 am Tibiateil 20 erfolgt innerhalb einer Lagerhülse 4 aus Kunststoff, insbesondere Polyethylen, wobei diese Lagerhülse 4 am Tibiateil 20 rotationsgesichert plaziert ist, nämlich innerhalb des sich distal erstreckenden Tibiagehäuses 3.

Der Chirurg kann noch in der Schlußphase der Operation entscheiden, ob er eine Lagerhülse 4 einsetzt, die entweder nur eine Rotationsbewegung des Lagerkörpers 1 erlaubt oder die sowohl eine Rotationsbewegung als auch Translationsbewegung in Richtung posterior-anterior gewährleistet. Auch kann der Operateur eine Auswahl aus einem Satz von Lagerhülsen treffen, deren langlochartige Ausnehmungen 24 in Richtung posterior-anterior unterschiedlich lang sind, mit der Folge, daß der Anschlag für die Translationsbewegung in Richtung anterior oder in Richtung posterior entsprechend unterschiedlich liegt.

Die Begrenzung der anterior-posterioren Translationsbewegung wird durch die anteriore und posteriore Begrenzung der langlochartigen Ausnehmung erhalten. Diese begrenzen die Kippbewegung des Lenkers.

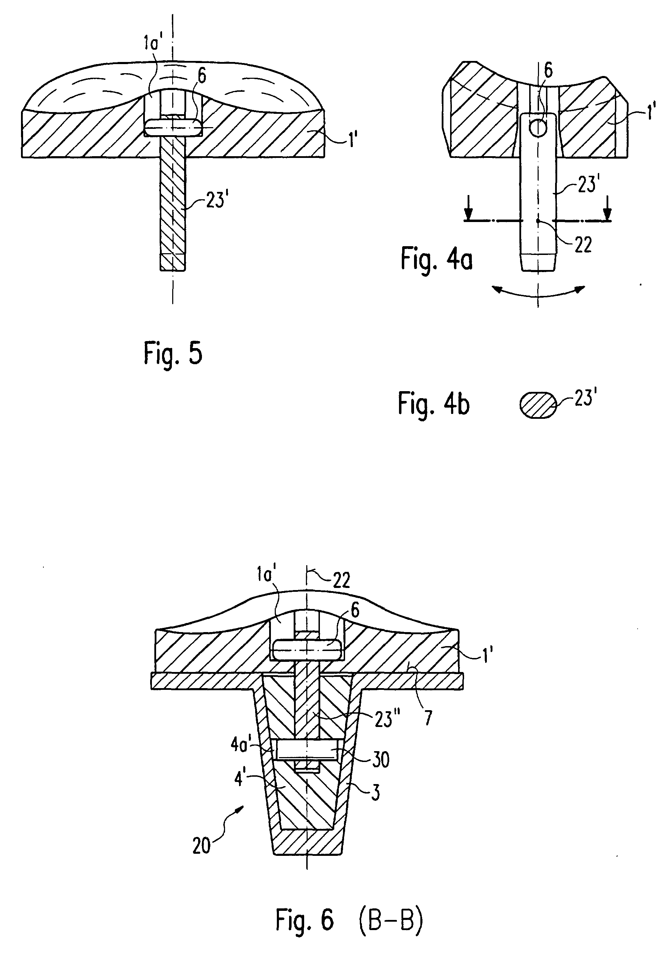

Die Ausführungsform nach den Figuren 4a bis 5 ist dadurch gekennzeichnet, daß am proximalen Ende eines Lenkers 23' eine sich in Richtung medial-lateral etwa parallel zur Tibialagerfläche 7 erstreckende Schwenkachse 6 angeordnet ist, die sich durch eine entsprechende Querbohrung im Lenker 23' hindurch erstreckt. Gemäß Fig. 4b ist der Querschnitt des Lenkers 23' abgeflacht. Dadurch erhält man in vorteilhafter Weise statt eines Linienkontaktes einen flächigen Kontakt zwischen dem Lenker 23' einerseits und den medialen und lateralen Begrenzungswänden der (nicht gezeigten) Lagerhülse andererseits.

Die Schwenkachse 6 greift in eine komplementär ausgebildete Schwenklagerausnehmung 1a' am Lagerkörper 1' ein. Diesbezüglich handelt es sich um eine übliche Konstruktionsmaßnahme, deren nähere Beschreibung sich hier erübrigt.

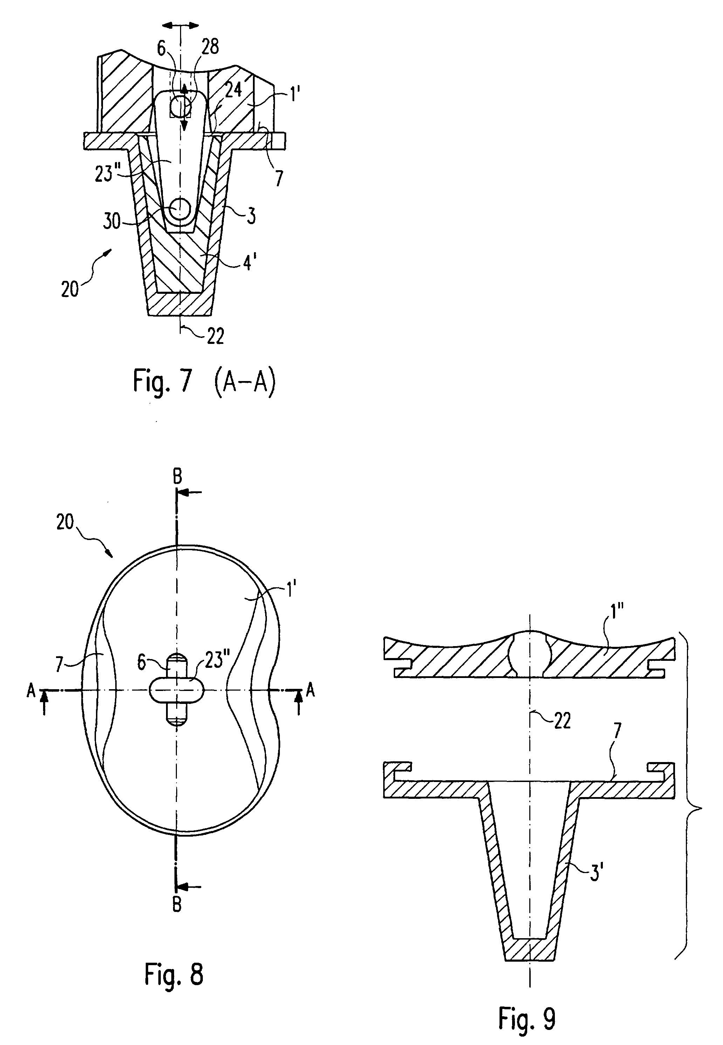

Die Ausführungsform gemäß den Figuren 6 bis 8 unterscheidet sich von derjenigen gemäß den Figuren 4a bis 5 dadurch, daß ein modifizierter Lenker 23'' mit seinem distalen Ende am Tibiateil 20 verschwenkbar gelagert ist, wobei diese distale und/oder die proximale Schwenklagerung am Lagerkörper 1' eine lenkeraxiale Translationsbewegung erlaubt, so daß eine dauerhafte Gleitauflage des Lagerkörpers 1' auf der Tibialagerfläche 7 unabhängig von der Schwenkstellung des Lenkers 23'' gewährleistet ist. Diese relative lenkeraxiale Translationsbewegung ist in Fig. 7 mit dem Doppelpfeil 28 angedeutet.

Das distale Schwenklager des Lenkers 23'' ist ebenfalls durch eine sich quer durch den Lenker 23'' hindurch erstreckende Schwenkachse 30 und eine komplementäre Aufnahmebohrung 4a' im Lagergehäuse 4' definiert, wobei sich die Schwenkachse 30 parallel zur Schwenkachse 6 im Lagerkörper 1' erstreckt.

Bei den Ausführungsformen nach Fig. 4 bis 8 ist der Lenker am Lagerkörper lediglich schwenkbar gelagert. Daher muß die Lagerhülse am Tibiateil um ihre Längsachse verdrehbar gelagert sein, um eine optimal anatomiegerechte Führung zu ermöglichen.

Die Ausführungsform nach Fig. 9 entspricht im wesentlichen derjenigen nach den Figuren 1 bis 3, wobei nur ein (modifizierter) Lagerkörper 1'' und ein modifiziertes Tibiagehäuse 3' im Längsschnitt in Richtung medial-lateral gezeigt sind. Diese Ausführungsform zeichnet sich dadurch aus, daß der Lagerkörper 1'' an der medialen und lateralen Seite durch das Tibiagehäuse 3' hintergriffen wird, so daß mit Sicherheit ein Abheben des Lagerkörpers 1 von der Tibialagerfläche 7 verhindert wird. Die den Lagerkörper 1 hintergreifenden Vorsprünge des Tibiagehäuses 3 haben posterior und anterior (nicht gezeigte) Anschläge, durch die die Rotation des Lagerkörpers 1 begrenzt wird. Dabei ist es möglich, lateral die Anschläge weiter voneinander zu beabstanden als medial mit der Folge, daß der Lagerkörper 1'' lateral eine größere Bewegungsfreiheit hat als medial. Diese unterschiedliche Bewegungsfreiheit entspricht dem natürlichen Bewegungsablauf eines Knies.

Die erwähnten Rotationsanschläge für den Lagerkörper an der medialen und lateralen Seite können natürlich auch unabhängig von den Maßnahmen zur Hintergreifung des Lagerkörpers an der medialen und lateralen Seite vorgesehen sein, d.h. bei allen hier beschriebenen Ausführungsformen.

Zu den Maßnahmen zur Hintergreifung des Lagerkörpers sei noch erwähnt, daß diese mit einem derartigen Spiel in medialer und lateraler Richtung erfolgt, daß der Lagerkörper sich ungehindert in der durch den Rotations-Translationslenker vorgegebenen Weise auf der Tibialagerfläche bewegen kann. Die erwähnten Maßnahmen dienen ausschließlich dazu, den Lagerkörper in gleitender Anlage auf der Tibialagerfläche zu halten.

Der Lagerkörper besteht in herkömmlicher Weise aus Kunststoff, vorzugsweise Polyethylen, während das Tibiagehäuse aus einer körperverträglichen Metalllegierung (Titanlegierung) hergestellt ist, ebenso wie der Lenker samt Schwenklagerkopf oder Schwenkachse.

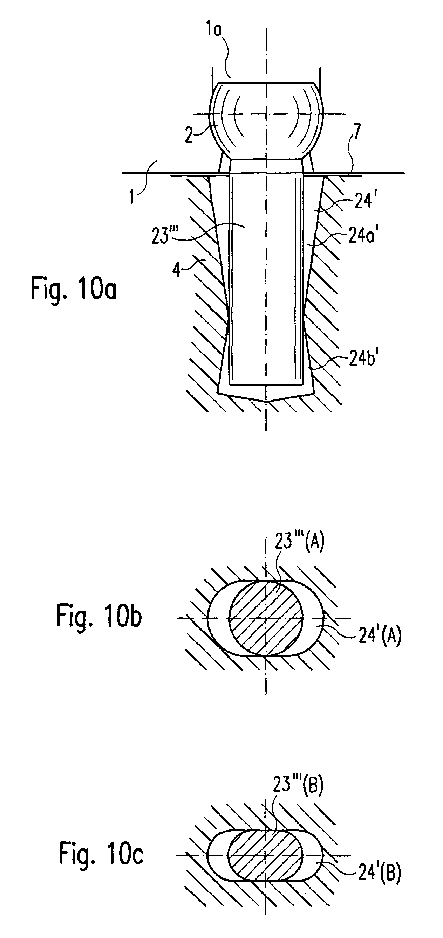

Die Figuren 10a bis 10c zeigen in einer Ausschnittdarstellung in Art einer Prinzipskizze eine weitere Ausführungsform der Erfindung, bei der ein Lenker 23''' mit zylindrischem Schaftabschnitt und kugelsegmentförmigem Schwenklagerkopf 2 in der Schwenklagerausnehmung 1a eines gemäß Fig. 1 bis 3 ausgebildeten Lagerkörpers 1 gehalten ist und mit dem Schaftabschnitt in eine Ausnehmung (ein Sackloch) 24' eingreift, das einen sich in posterior-anterior Richtung nach oben (proximal) trapezförmig erweiternden ersten Abschnitt 24a' sowie einen sich nach unten (distal) ebenfalls im wesentlichen trapezförmig erweiternden zweiten Abschnitt 24b' umfaßt. Durch diese Ausbildung der Ausnehmung 24' wird erreicht, daß der Lenker 23''' um eine in Höhe der engsten Stelle der Ausnehmung 24' liegende Schwenkachse zwängungsfrei in posterior-anterior Richtung schwenkbar ist.

In Fig. 10b und 10c sind Querschnittsformen für den Schaft des Lenkers 23''' und die Ausnehmung 24' (in Höhe der Tibialagerfläche 7) skizziert. Entsprechend der Zuordnung beider Varianten zu Fig. 10a sind die Teile hier mit den Bezugsziffern 23'''(A) bzw. 23'''(B) sowie 24'(A) und 24'(B) bezeichnet.

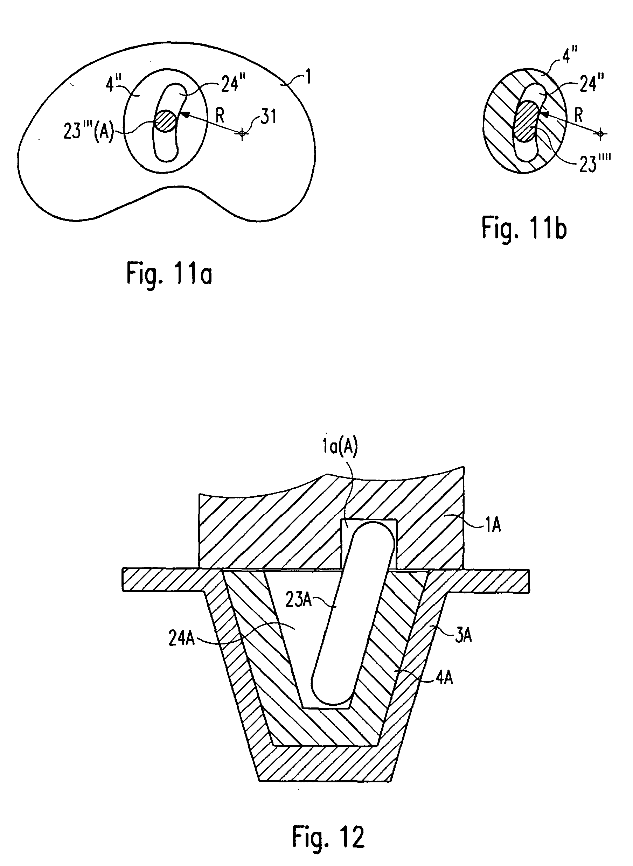

Die Fig. 11a und 11b zeigen in Prinzipskizzen eine weitere Ausführungsform, bei der unter dem Lagerkörper 1 eine Lagerhülse 4'' mit einer gebogenen Ausnehmung 24'' vorgesehen ist, in der ein Lenker 23'''(A) mit kreisförmigem Schaftquerschnitt (Fig. 11a) bzw. ein Lenker 23'''' mit an die Krümmung der Ausnehmung 24'' angepaßtem Schaftquerschnitt geführt ist. Die Ausnehmung 24'' ist um einen auf der medialen Seite liegenden Krümmungsmittelpunkt 31 mit einem Krümmungsradius R derart gekrümmt, daß sie nach medial hin konkav, d. h. nach lateral hin konvex, ausgebildet ist. Bei der Ausführung nach Fig. 11b gilt dies ebenso für den Schaftquerschnitt des Lenkers 23''''.

Fig. 12 ist eine vereinfachte Prinzipskizze zur Erläuterung der wesentlichen Funktionskomponenten und grundsätzlichen Funktionsweise der vorgeschlagenen Anordnung, der zufolge die Anordnung einen Lagerkörper 1A mit einer darin ausgebildeten Schwenklagerausnehmung 1a(A), ein Tibiagehäuse 3A mit Lagerhülse 4A und einer darin ausgebildeten Ausnehmung 24A und einen Lenker 23A umfaßt. Die hier skizzenartig dargestellte Führung würde grundsätzlich einer Luxationsgefahr unterliegen, so daß die Darstellung diesbezüglich als stark vereinfacht anzusehen ist. Sie zeigt aber das Prinzip der Drehführung des Lagerkörpers 1A durch den Lenker 23A, der relativ zum Lagerkörper schwenkbar gehalten und medial-laterial geführt ist.

### Bezugszeichenliste

- 1; 1'; 1''; 1A: Lagerkörper
- 1a; 1a'; 1a(a): Schwenklagerausnehmung
- 2: Schwenklagerkopf
- 3; 3'; 3A: Tibiagehäuse
- 4; 4'; 4''; 4A: Lagerhülse
- 4a': Aufnahmebohrung
- 6, 30: Schwenkachse
- 7: Tibialagerfläche
- 8: Lagerschale
- 20: Tibiateil
- 21, 27, 28, 29: Doppelpfeil
- 22: Drehachse
- 23; 23'; 23''; 23'''; 23'''(A); 23'''(B); 23A: Lenker
- 24; 24'; 24'(A); 24'(B); 24''; 24A: langlochartige Ausnehmung (Sackloch)
- 24a', 24b': Ausnehmungsabschnitte
- 25, 26: Begrenzungswand
- 31: Krümmungsmittelpunkt
- R: Krümmungsradius

## Patentansprüche

1. Kniegelenkendoprothese mit einem Tibiateil (20) mit ebener Tibialagerfläche (7), einem auf dieser in Richtung anterior-posterior verschiebbaren Lagerkörper (1; 1'; 1''; 1A) mit zwei konkav gewölbten Lagerschalen (8) zur beweglichen Aufnahme eines Femurgelenkteils und mit einer Drehführung, die eine Rotation des Lagerkörpers auf der Tibialagerfläche (7) um eine senkrecht auf dieser stehende Drehachse (22) erlaubt,
**dadurch gekennzeichnet,daß**
die Drehführung einen Lenker (23; 23'; 23''; 23'''; 23'''(A); 23'''(B); 23''''; 23A) umfaßt, der am Lagerkörper (1) relativ zu diesem schwenkbar und am Tibiateil (20) medial-lateral geführt und/oder drehgeführt ist.

2. Kniegelenkendoprothese nach Anspruch 1,
**dadurch gekennzeichnet, daß**
der Lenker (23; 23'; 23''; 23'''; 23'''(A); 23'''(B); 23''''; 23A) einerseits am Tibiateil (20) innerhalb einer sich in Richtung anterior-posterior erstreckenden langlochartigen Ausnehmung (24; 24'; 24'(A), 24'(B); 24''; 24A) medial und lateral geführt ist und anderseits entweder am Lagerkörper (1; 1'; 1''; 1A) relativ zu diesem sowohl dreh- als auch schwenkbar gehalten oder am Lagerkörper nur schwenkbar gehalten und zusammen mit der Ausnehmung im Tibiateil (20) um eine senkrecht auf der Tibialagerfläche (7) stehende Drehachse (22) drehbar gelagert ist.

3. Kniegelenkendoprothese nach Anspruch 2,
**dadurch gekennzeichnet, daß**
die langlochartige Ausnehmung (24; 24'; 24'(A), 24' (B); 24''; 24A ) als Sackloch ausgebildet ist, das sich zur Tibialagerfläche (7) hin mindestens im oberen Bereich von distal nach proximal trapezförmig erweitert sowie medial und lateral durch sich parallel zueinander und etwa senkrecht zur Tibialagerfläche (7) erstreckende Wände (25, 26) begrenzt ist, deren Abstand voneinander für eine Spielpassung geringfügig größer ist als die entsprechende Abmessung des in der Ausnehmung aufgenommenen Lenkers (23; 23'; 23''; 23'''; 23'''(A); 23'''(B); 23''''; 23A) bzw. Lenkerabschnitts.

4. Kniegelenkendoprothese nach Anspruch 3,
**dadurch gekennzeichnet, daß**
das Sackloch (24'; 24'(A), 24'(B)) sich in Richtung anterior-posterior im unteren Bereich von der Tibialagerfläche weg trapezförmig erweitert derart, daß eine im wesentlichen zwängungsfreie Verschwenkung des Lenkers (23'''; 23'''(A); 23'''(B)) um eine von seinem distalen Ende beabstandete Drehachse gewährleistet ist.

5. Kniegelenkendoprothese nach einem der vorangehenden Ansprüche,
**dadurch gekennzeichnet, daß**
der Lenker (23; 23'''; 23'''(A), 23'''(B)) am proximalen Ende einen kugelartigen Kopf (2) oder eine sich in Richtung medial-lateral im wesent-lichen parallel zur Tibialagerfläche (7) erstreckende Schwenkachse (6) umfaßt, der bzw. die in eine komplementär ausgebildete Schwenklagerausnehmung am Lagerkörper (1) eingreift.

6. Kniegelenkendoprothese nach einem der Ansprüche 1 bis 3,
**dadurch gekennzeichnet, daß**
die Führung des Lenkers (23; 23'''; 23'''(A); 23'''(B)) am Tibiateil (20) innerhalb einer Lagerhülse (4; 4'; 4A) aus Kunststoff, insbesondere Polyethylen, erfolgt, wobei diese Lagerhülse am Tibiateil rotationsgesichert plaziert ist.

7. Kniegelenkendoprothese nach Anspruch 6,
**dadurch gekennzeichnet, daß**
die Lagerhülse (4; 4'; 4A) in einem Tibiagehäuse (3; 3A) aufgenommen ist, welches ein sich zur Tibialagerfläche (7) hin erweiterndes Sackloch zum Einsetzen der Lagerhülse aufweist.

8. Kniegelenkendoprothese nach einem der vorangehenden Ansprüche,
**dadurch gekennzeichnet, daß**
die langlochartige Ausnehmung (24'') eine, insbesondere kreisbogenförmige und durch einen Krümmungsradius (R) um einen medial der Ausnehmung liegenden Krümmungsmittelpunkt (31) bestimmte, Krümmung aufweist derart, daß sie nach medial eine konkave bzw. nach lateral eine konvexe Gestalt hat.

9. Kniegelenkendoprothese nach einem der vorangehenden Ansprüche,
**dadurch gekennzeichnet, daß**
der Lenker (23; 23'''; 23'''(A); 23'''(B)) oder mindestens der in der langlochartigen Ausnehmung aufgenommene Lenkerabschnitt mindestens zwei zylinder- oder kegelstumpfsegmentförmige Wandungsabschnitte aufweist.

10. Kniegelenkendoprothese nach Anspruch 8 und Anspruch 9,
**dadurch gekennzeichnet, daß**
der Lenker (23'''') bzw. der in der langlochartigen Ausnehmung (24; 24'; 24'(A); 24'(B); 24''; 24A) aufgenommene Lenkerabschnitt bei gekrümmter Ausbildung der Ausnehmung (24'') im wesentlichen zylinder- oder kegelstumpfförmig ist oder zwei die zylinder- oder kegelstumpfsegmentförmigen Abschnitte verbindende, in Anpassung an die Krümmung der Ausnehmung gekrümmte Wandungsabschnitte hat.

11. Kniegelenkendoprothese nach Anspruch 9,
**dadurch gekennzeichnet, daß**
der Lenker (23'; 23''; 23''') bzw. der in der langlochartigen Ausnehmung aufgenommene Lenkerabschnitt bei einer Ausführung der Ausnehmung (24; 24'; 24'(A); 24'(B)) mit ebenen Wandungsabschnitten zwei die zylinder- oder kegelstumpfsegmentförmigen Abschnitte verbindende ebene Wandungsabschnitte aufweist.

12. Kniegelenkendoprothese nach einem der Ansprüche 1 bis 4,
**dadurch gekennzeichnet, daß**
der Lenker (23) mit einem distalen Ende am Tibiateil (20) verschwenkbar gelagert ist, wobei diese distale und/oder die proximale Schwenklagerung am Lagerkörper (1) eine lenkeraxiale Translationsbewegung (28) erlaubt, so daß eine dauerhafte Gleitauflage des Lagerkörpers (1) auf der Tibialagerfläche (7) unabhängig von der Schwenkstellung des Lenkers (23) gewährleistet ist.

13. Kniegelenkendoprothese nach einem der vorangehenden Ansprüche,
**dadurch gekennzeichnet, daß**
das im Lagerkörper (1; 1'') aufgenommene und/oder das am Tibiateil geführte Ende des Lenkers (23; 23'''') im wesentlichen kugelabschnittförmig abgerundet ist.

## Claims

1. Knee joint endoprosthesis having a tibial component (20) having a flat tibial bearing surface (7), a bearing body (1; 1'; 1"; 1A) which is displaceable thereon in the anterior-posterior direction and which has two concavely curved bearing shells (8) for movably accommodating a femoral joint component, and having a rotary guide means which allows rotation of the bearing body on the tibial bearing surface (7) about an axis of rotation (22) extending perpendicular thereto,
**characterised in that**
the rotary guide means comprises a linker (23; 23'; 23"; 23'''; 23"'(A); 23'''(B); 23""; 23A) which is guided at the bearing body (1) so that it can pivot relative thereto and which, at the tibial component (20), is guided medially-laterally and/or rotatably guided.

2. Knee joint endoprosthesis according to claim 1,
**characterised in that**
the linker (23; 23'; 23"; 23'''; 23"'(A); 23'''(B); 23""; 23A) on the one hand is guided at the tibial component (20) medially and laterally within an elongate-hole-shaped recess (24; 24'; 24'(A), 24'(B); 24"; 24A) extending in the anterior-posterior direction and on the other hand either is held at the bearing body (1; 1'; 1"; 1A) so that it can both rotate and pivot relative thereto or is held at the bearing body so that it can only pivot and is mounted so that together with the recess in the tibial component (20) it can rotate about an axis of rotation (22) extending perpendicular to the tibial bearing surface (7).

3. Knee joint endoprosthesis according to claim 2,
**characterised in that**
the elongate-hole-shaped recess (24; 24'; 24'(A), 24'(B); 24"; 24A) is in the form of a blind hole which widens out trapezoidally in the distal-to-proximal direction towards the tibial bearing surface (7) at least in the upper region and which is bounded medially and laterally by walls (25, 26) extending parallel to one another and approximately perpendicular to the tibial bearing surface (7), the spacing between which walls is, for a fit with play, slightly greater than the corresponding dimension of the linker (23; 23'; 23"; 23'''; 23'''(A); 23'''(B); 23""; 23A) or linker portion accommodated in the recess.

4. Knee joint endoprosthesis according to claim 3,
**characterised in that**
the blind hole (24'; 24'(A), 24'(B)) widens out trapezoidally in the anterior-posterior direction in the lower region away from the tibial bearing surface in a manner ensuring substantially unconstrained pivoting of the linker (23'''; 23'''(A); 23'''(B)) about an axis of rotation spaced away from its distal end.

5. Knee joint endoprosthesis according to one of the preceding claims,
**characterised in that**
the linker (23; 23'''; 23'''(A); 23'''(B)) comprises at its proximal end a ball-like head (2) or, extending in the medial-lateral direction substantially parallel to the tibial bearing surface (7), a pivot pin (6), which head or pin engages in a complementarily formed pivot mounting recess in the bearing body (1).

6. Knee joint endoprosthesis according to one of claims 1 to 3,
**characterised in that**
the guidance of the linker (23; 23'''; 23"'(A); 23'''(B)) at the tibial component (20) takes place within a bearing sleeve (4; 4'; 4A) made from plastics material, especially polyethylene, that bearing sleeve being located at the tibial component in a manner preventing its rotation.

7. Knee joint endoprosthesis according to claim 6,
**characterised in that**
the bearing sleeve (4; 4'; 4A) is accommodated in a tibial housing (3; 3A) which, for insertion of the bearing sleeve, has a blind hole widening out towards the tibial bearing surface (7).

8. Knee joint endoprosthesis according to one of the preceding claims,
**characterised in that**
the elongate-hole-shaped recess (24") has a curve, especially a curve in the shape of an arc of a circle and defined by a curve radius (R) about a curve centrepoint (31) located medial to the recess, so that it has a medially concave and laterally convex shape.

9. Knee joint endoprosthesis according to one of the preceding claims,
**characterised in that**
the linker (23; 23'''; 23'''(A); 23'''(B)) or at least the linker portion accommodated in the elongate-hole-shaped recess has at least two wall portions of cylindrical or truncated cone segment shape.

10. Knee joint endoprosthesis according to claim 8 and claim 9,
**characterised in that**
the linker (23"") or the linker portion accommodated in the elongate-hole-shaped recess (24; 24'; 24'(A); 24'(B); 24"; 24A), in the case where the recess (24") is curved, is of substantially cylindrical or truncated cone shape or has two wall portions which connect the portions of cylindrical or truncated cone segment shape and which are curved in conformity with the curve of the recess.

11. Knee joint endoprosthesis according to claim 9,
**characterised in that**
the linker (23'; 23"; 23''') or the linker portion accommodated in the elongate-hole-shaped recess, in the case where the recess (24; 24'; 24'(A); 24'(B)) has flat wall portions, has two flat wall portions which connect the portions of cylindrical or truncated cone segment shape.

12. Knee joint endoprosthesis according to one of claims 1 to 4,
**characterised in that**
the linker (23) is, at a distal end, pivotally mounted at the tibial component (20), that distal pivot mounting and/or the proximal pivot mounting at the bearing body (1) allowing translational movement (28) along the axis of the linker so that it is ensured that the bearing body (1) remains in sliding contact with the tibial bearing surface (7) whatever the pivoted position of the linker (23).

13. Knee joint endoprosthesis according to one of the preceding claims,
**characterised in that**
the end of the linker (23; 23"") accommodated in the bearing body (1; 1") and/or guided at the tibial component is rounded off substantially in the shape of part of a sphere.

## Revendications

1. Endoprothèse de l'articulation du genou avec une partie tibiale (20) munie d'une surface d'appui tibiale (7) plane, un palier (1 ; 1' ; 1 " ; 1A) mobile sur cette dernière dans la direction antérieure-postérieure, avec deux coquilles de support (8) à courbure concave, destinées à recevoir une partie d'articulation fémorale et avec un guidage rotatif, qui permet une rotation du palier sur la surface d'appui tibiale (7) autour d'un axe de rotation (22) perpendiculaire à cette dernière, **caractérisée en ce que** le guidage rotatif comporte un bras d'orientation (23 ; 23' ; 23" ; 23''' ; 23"'(A) ; 23"'(B) ; 23"" ; 23A), qui est guidé au niveau du palier (1) de manière pivotante par rapport à ce dernier et est guidé de médial à latéral au niveau de la partie formant tibia (20) et/ou guidé en rotation.

2. Endoprothèse de l'articulation du genou selon la revendication 1, **caractérisée en ce que** le bras d'orientation (23 ; 23' ; 23" ; 23''' ; 23'''(A) ; 23'''(B) ; 23"" ; 23A), d'une part, est guidé au niveau de la partie formant tibia (20) dans la zone médiale et latérale à l'intérieur d'un évidement (24 ; 24' ; 24'(A) ; 24'(B) ; 24" ; 24A) qui s'étend en forme de trou oblong dans la direction antérieure-postérieure et, d'autre part, est maintenu au niveau du palier (1 ; 1' ; 1" ; 1A) de manière rotative et pivotante par rapport à celui-ci ou est maintenu seulement de manière pivotante contre le palier et, conjointement avec l'évidement dans la partie formant tibia (20), est logé de manière rotative autour d'un axe de rotation (22) perpendiculaire à la surface d'appui tibiale (7).

3. Endoprothèse de l'articulation du genou selon la revendication 2, **caractérisée en ce que** l'évidement (24 ; 24' ; 24'(A) ; 24'(B) ; 24" ; 24A) en forme de trou oblong est réalisé sous forme de trou borgne, qui s'élargit avec une forme trapézoïdale vers la surface d'appui tibiale (7), au moins dans la partie supérieure de distal à proximal, et qui est délimité dans la zone médiale et latérale par des parois (25, 26) qui sont parallèles l'une à l'autre et perpendiculaires à la surface d'appui tibiale (7) et qui, pour former un logement avec jeu, sont écartées l'une de l'autre sur une distance légèrement supérieure à la dimension correspondante du bras d'orientation (23 ; 23' ; 23" ; 23''' ; 23"'(A) ; 23'''(B) ; 23"" ; 23A) ou d'un tronçon du bras d'orientation, logé dans l'évidement.

4. Endoprothèse de l'articulation du genou selon la revendication 3, **caractérisée en ce que** le trou borgne (24' ; 24'(A), 24'(B)) s'élargit avec une forme trapézoïdale dans la direction antérieure-postérieure dans la partie inférieure en s'écartant de la surface d'appui tibiale, de manière à garantir un pivotement sensiblement non forcé du bras d'orientation (23''' ; 23'''(A) ; 23'''(B)) autour d'un axe de rotation écarté de son extrémité distale.

5. Endoprothèse de l'articulation du genou selon l'une quelconque des revendications précédentes, **caractérisée en ce que** le bras d'orientation (23 ; 23''' ; 23'''(A) ; 23'''(B)) comporte sur son extrémité proximale une tête (2) sphérique ou un axe de pivotement (6) qui s'étend dans la direction médiale-latérale sensiblement parallèlement à la surface d'appui tibiale (7), laquelle tête ou lequel axe de pivotement s'engagent dans un évidement formant logement de pivotement, réalisé de manière complémentaire dans le palier (1).

6. Endoprothèse de l'articulation du genou selon l'une quelconque des revendications 1 à 3, **caractérisée en ce que** le guidage du bras d'orientation (23 ; 23''' ; 23"'(A) ; 23'''(B)) dans la partie formant tibia (20) est assuré à l'intérieur d'une douille de support (4 ; 4' ; 4A) en matière plastique, en particulier en polyéthylène, ladite douille de support étant positionnée de manière bloquée en rotation dans la partie formant tibia (20).

7. Endoprothèse de l'articulation du genou selon la revendication 6, **caractérisée en ce que** la douille de support (4 ; 4' ; 4A) est logée dans un boîtier du tibia (3 ; 3A), qui comporte un trou borgne qui s'élargit vers la surface d'appui tibiale (7) et est destiné à recevoir la douille de support.

8. Endoprothèse de l'articulation du genou selon l'une quelconque des revendications précédentes, **caractérisée en ce que** l'évidement (24") en forme de trou oblong est réalisé avec une courbure, en particulier en forme d'arc de cercle et déterminée par un rayon de courbure (R) autour d'un centre de courbure (31) situé du côté médial par rapport à l'évidement, de telle sorte que ledit évidement présente vers la zone médiale une forme concave et vers la zone latérale une forme convexe.

9. Endoprothèse de l'articulation du genou selon l'une quelconque des revendications précédentes, **caractérisée en ce que** le bras d'orientation (23 ; 23''' ; 23"'(A) ; 23'''(B)) ou au moins le tronçon du bras d'orientation, logés dans l'évidement en forme de trou oblong, comportent au moins deux parties de parois cylindriques ou en forme de segment tronconique.

10. Endoprothèse de l'articulation du genou selon les revendications 8 et 9, **caractérisée en ce que**, dans le cas d'un évidement (24") courbe, le bras d'orientation (23"") ou le tronçon du bras d'orientation, logés dans l'évidement (24 ; 24' ; 24'(A) ; 24'(B) ; 24" ; 24A) en forme de trou oblong, sont sensiblement cylindriques ou tronconiques ou comportent deux parties de paroi, qui relient les tronçons cylindriques ou en forme de segment tronconique et ont une courbure adaptée à la courbure de l'évidement.

11. Endoprothèse de l'articulation du genou selon la revendication 9, **caractérisée en ce que**, dans le cas d'un évidement (24 ; 24' ; 24'(A) ; 24'(B)) conçu avec des parties de paroi planes, le bras d'orientation (23' ; 23" ; 23"') ou le tronçon du bras d'orientation, logés dans l'évidement en forme de trou oblong, comportent deux parties de paroi planes, qui relient les tronçons cylindriques ou en forme de segment tronconique.

12. Endoprothèse de l'articulation du genou selon l'une quelconque des revendications 1 à 4, **caractérisée en ce que** le bras d'orientation (23) est logé pivotant avec une extrémité distale contre la partie formant tibia (20), cette extrémité distale et/ou le logement de pivotement proximal dans le palier (1) permet un mouvement de translation (28) dans le sens axial du bras d'orientation, de manière à garantir un appui par glissement permanent du palier (1) sur la surface d'appui tibiale (7) indépendamment de la position de pivotement du bras d'orientation (23).

13. Endoprothèse de l'articulation du genou selon l'une quelconque des revendications précédentes, **caractérisée en ce que** l'extrémité du bras d'orientation (23 ; 23""), logée dans le palier (1 ; 1") et/ou guidée au niveau de la partie formant tibia (20), est sensiblement arrondie avec une forme partiellement sphérique.
